(19) Europäisches Patentamt
European Patent Office
Office européen des brevets

(11) Publication number: **0 314 184 B1**

# EUROPEAN PATENT SPECIFICATION

(45) Date of publication of patent specification: **13.12.95**

(51) Int. Cl.⁶: **C12N 15/70**, C12N 15/67

(21) Application number: **88118135.8**

(22) Date of filing: **31.10.88**

(54) **Expression plasmids**

(30) Priority: **30.10.87 US 115139**

(43) Date of publication of application:
**03.05.89 Bulletin 89/18**

(45) Publication of the grant of the patent:
**13.12.95 Bulletin 95/50**

(84) Designated Contracting States:
**ES GR**

(56) References cited:
EP-A- 0 099 084        EP-A- 0 170 266
EP-A- 0 196 864        EP-A- 0 200 590
WO-A-83/04052          WO-A-85/03522

**CHEMICAL ABSTRACTS, vol. 107, no. 1, 6th July 1987, page 189, abstract no. 18847u, Columbus, Ohio, USA; RONNY LEEMANS et al.**

**BIOTECHNOLOGY, vol. 5, no. 6, June 1987, pages 600-603, New York, NY, USA; H.J. GEORGE et al.**

**THE EMBO JOURNAL, vol. 6, no. 3, March 1987, pages 823-831, Eynsham, Oxford, Gr. Britain; M. SJÖSTRÖM et al.**

**GENE, vol. 60, no. 2-3, 1987, pages 175-182, Amsterdam, NC; A.F. PURCHIO et al.**

(73) Proprietor: **Bristol-Myers Squibb Company 345 Park Avenue New York, N.Y. 10154 (US)**

(72) Inventor: **Liu, Suo Win 225 Marsh Drive DeWitt, New York 13214 (US)**
Inventor: **Bruce, A. Gregory 2433 36 West, Seattle Washington 98199 (US)**
Inventor: **Franceschini, Thomas J. 406 Wembridge Drive East Syracuse, New York 13057 (US)**
Inventor: **Rose, Timothy M. 326 NE 59th Street, Seattle Washington 98105 (US)**

(74) Representative: **Kinzebach, Werner, Dr. Patentanwälte Reitstötter, Kinzebach und Partner Postfach 86 06 49 D-81633 München (DE)**

EP 0 314 184 B1

**Description**

BACKGROUND OF THE INVENTION

Field of the Invention

This invention relates to expression plasmids, or expression vectors, useful for expressing foreign genes by way of recombinant DNA technology.

Background Art

The use of the bacteriophage lambda promoter $P_L$ and the cI repressor to regulate the expression of foreign genes is known. U. S. Patent 4,530,901 (1985) to Weissman discloses recombinant DNA molecules and their use in producing human interferon-like polypeptides. In one embodiment, the patent teaches that the expression control sequence can be selected from the major operator and promoter regions of phage lambda, including $O_L/P_L$ (col. 32).

PCT Application WO 83/00092 (1983) discloses an expression vector comprising the origin of replication derived from a bacterial plasmid as for example pBR322, a promoter from bacteriophage lambda, as for example $P_L$, a region for the initiation of translation encoding, for example, the repressor cI derived from bacteriophage lambda cI 857S7, a DNA sequence for the lambda N gene, a selectable marker, and a cloning site for a foreign gene downstream from the $P_L$ promoter.

U. S. Patent 4,520,103 (1985) to Ensley, directed to the microbial production of indigo, discloses the use of the $P_L$ promoter under the control of the cI857 repressor for regulating the expression of genes cloned on plasmids described in the patent.

European Patent Application 0119621 (1984), directed to the expression of interleukin-2, discloses expression vectors in which the gene is under the control of $O_L/P_L$ and in which the promoter is regulated by the cI857 repressor.

European Patent Application 0131843 (1985) describes vectors for the expression of polypeptides. The vector comprises the $O_L/P_L$ regulatory region, the N utilization site for binding antiterminator N protein produced by the host cell, a ribosomal binding site, ATG initiation codon and restriction enzyme recognition site for inserting the gene of interest. The vector does not contain the cI repressor; the repressor is contained in the host cells transformed by the vector.

European Patent Application 0041767 (1981) discloses vectors and methods for expressing cloned genes. The vectors are characterized by a promoter and operator from bacteriophage lambda and a restriction enzyme recognition site located less than 300 base pairs away from that regulatory region.

PCT Application WO85/00829 (1985) discloses novel plasmids for the expression of exogenous genes. In one embodiment, the promoter is the lambda promoter $P_L$ and the vector also includes a thermally responsive repressor which is functionally associated with the promoter.

PCT Application WO85/03522 (1985) concerns monitoring and control systems for recombinant manipulations. The application describes portable control cassettes comprising the $P_L$ promoter which is controllable by means of a temperature sensitive repressor, operably linked to the ribosomal binding site for N gene. Downstream of this site is a restriction site allowing the insertion of the gene of interest. A translation initiation codon may be present within the cassette directly upstream of this site or the ATG may be contained on the gene of interest. The ultimate focus of this application is on a novel universal dominant selectable marker cassette comprising a modified and truncated coding sequence of aminoglycoside phosphotransferase (mtAPH-I) and on fusion proteins having C-terminal sequences comprising the amino acids encoded by MtAPH-I.

U.S. Patent 4,436,815 (1984) to Hershberger has as an object the use of a plasmid-borne repressor to repress a host cell lethal gene so that only those cells carrying the plasmid will survive. One such plasmid (see col. 4) contains a cI857 repressor on a 2.5 kb BglII fragment of bacteriophage lambda DNA. The patent discloses the uses of a variety of promoters, but not $P_L$, for the patent teaches that promoters sensitive to the repressor should not be used.

U.S. Patent 4,582,800 (1986) to Crowl discloses an expression vector, which is a derivative of pBR322, comprising a DNA sequence which comprises the $P_L$ promoter and $O_L$ operator derived from bacteriophage lambda, a hybrid ribosome binding site, cI isolated from a lambda phage which codes for a temperature sensitive repressor protein, and a eucaryotic gene which codes for interferon, preferably human immune interferon, an organism, such as E. coli bacteria, transformed by this vector, and a method for producing interferon.

U.S. Patent 4,543,329 (1985) relates a process for cleaving proteins such as fusion proteins via a cleavage site genetically engineered at the linkage point between the native protein sequence and the foreign protein which is susceptible to specific enzymatic cleavage.

U.S. Patent 4,565,785 (1986) relates to a process for producing foreign proteins in bacteria by inserting the foreign gene into that of a periplasmic or extracellular protein such that the foreign protein is excreted from the bacterial cell.

European Patent Application 0161937 (1985) relates a process for the production of a desired protein in native form where the desired protein is expressed as part of a fusion protein containing a Factor Xa enzymatic cleavage site which upon cleavage with Factor Xa yields the foreign gene product in native form.

U.S. Patent 4,624,926 (1986) relates to a class of recombinant bacterial plasmid cloning vehicles for expression of exogenous genes in transformed bacterial hosts where the exogenous gene is linked to a functional fragment derived from an outer membrane protein gene of any gram-negative bacterium.

European Patent Application 0173254 (1985) relates a DNA sequence of the EBV-genome that contains at its 5′ end an oligonucleotide coding for an oligopeptide which serves as a cleavage site for a sequence specific protease or which is cleavable by acid treatment.

There remains a need for a bacterial expression vector giving the advantages of a system wherein a foreign gene may be expressed to high levels under tight transcriptional control where the resulting gene product is able to accumulate without degradation, and be conveniently processed and renatured to a functional state.

We have devised an expression vector which yields the above advantages. The present invention has nucleotide sequences of the strong lambda bacteriophage $P_L$ promoter and $O_L$ operator along with the temperature sensitive cI repressor, a system which allows the controlled activation of transcription at $P_L$ by temperature. The present invention also has the nucleotide sequences of the ribosomal binding site of the lambda N gene and the nucleotide sequences coding for the amino-terminal amino acids of a highly expressed gene followed by a restriction site to allow the insertion of a foreign gene. At 30°C, transcription from the $P_L$ promoter is repressed, and bacteria containing this system multiply unhindered by the production and accumulation of a foreign gene product which potentially may be toxic. When the bacteria have reached an optimal concentration, the temperature is increased to 42°C at which time the cI repressor is rendered inactive and transcription at the $P_L$ promoter allows high level expression of the foreign gene.

Optimal ribosome binding and initiation of translation is achieved in one case by utilizing the bacteriophage lambda N gene ribosomal binding site and the nucleotide sequences coding for the amino-terminal amino acids of the highly expressed lambda N protein. This combination allows for efficient translation of the messenger RNA yielding large amounts of a fusion protein containing amino acids of the N protein at the amino terminus and the amino acids of the foreign protein at the carboxy terminus. The present invention also includes the nucleotide sequences coding for the acid labile dipeptide aspartic acid-proline which is positioned between the nucleotide sequences coding for the amino-terminal amino acids of the lambda N protein and those sequences coding for the foreign gene. After purification of the highly expressed fusion protein, acid treatment will liberate the foreign gene product.

In another case, the N gene ribosomal binding site is followed by the nucleotide sequences coding for a hydrophobic signal sequence where the amino acids for the signal sequence were designed to resemble the signal sequence amino acids of the E. coli pho A protein. Furthermore, nucleotides were designed to resemble closely the nucleotides of the amino-terminal amino acids of the lambda N protein. The presence of the hydrophobic signal sequence allows the foreign gene product to be highly expressed as part of a larger precursor polypeptide and subsequently to be specifically cleaved proteolytically from the signal sequence. In the case described hereinafter, the heterologous proteins expressed appear to be compartmentalized in an aggregated form within the bacteria rather than secreted as soluble periplasmic proteins.

Expression of the foreign gene product as part of a fusion with another polypeptide, as described in the present invention, has several beneficial aspects. It aids in the high level expression of foreign gene products and helps prevent the foreign gene products from being degraded in vivo. It is useful in the expression of small foreign proteins which are especially susceptible to degradation in the bacterial host. The presence of the amino terminus of the N protein is useful in that it allows for general methods for the purification of any foreign protein in such a fusion product. The amino terminal amino acids of the N protein are predicted to be antigenic and specific antibodies toward N gene can be used for the immunopurification of fusion proteins. In addition, the highly positive charged nature of the amino terminus of the N protein could be utilized in purification schemes. The charged nature of the amino terminus of the N protein will allow the fused protein to be more easily solubilized during purification even if the foreign protein is in an unfolded denatured and thus more insoluble state. The charged nature of the amino terminus may help the renaturation of the foreign protein in subsequent manipulations to regain activity. The presence of several lysines in the amino terminus of such fusion proteins could be utilized to chemically couple the fusion protein to support matrices for a variety of applications. Furthermore, in our case, the "authentic" foreign proteins can be obtained from the expressed heterologous proteins by treatment of the fusion proteins with cyanogen bromide if the expressed foreign genes do not contain internal methionine residue(s). Moreover, an acid labile site or specific protein cleavage site(s) can be inserted between the sequence coding for N gene and the sequences coding for the foreign gene(s). "Authentic" foreign protein can be released from the fusion proteins by treatment of the expressed heterologous fusion protein with either acid or specific proteases, respectively. The presence of the cI repressor on the vector allows the vector to be transformed into any bacteria host which does not have the repressor gene integrated into its chromosome. The presence of antibiotic markers (i.e., neomycin or tetracycline) will be useful for large scale production by stabilizing the plasmid.

## SUMMARY OF THE INVENTION

This invention comprises expression vectors, particularly a set of E. coli expression vectors which have been constructed for expressing foreign genes. All of the vectors contain the origin of replication derived from pBR322, the temperature inducible strong leftward promoter ($P_L$) derived from bacteriophage lambda, DNA sequences coding for the thermolabile repressor cI derived from bacteriophage lambda cI857S7, and DNA sequences for a ribosomal binding site, particularly the ribosomal binding site of the lambda N gene or the E. coli consensus ribosomal binding site. Optionally, some vectors according to this invention further contain DNA sequences coding for the amino-terminal amino acids of a highly expressed bacteriophage gene. Plasmid pBM11 and plasmid pBM14 contain the nucleotide sequence coding for the first 33 amino acids of the bacteriophage lambda N protein. Plasmid and pBM11 contains the the neomycin resistance gene as a selective marker whereas plasmid pBM14 contains the tetracycline resistance gene. Plasmid pBM11 contains a unique BamH I site for cloning of a foreign gene downstream from the $P_L$ promoter and plasmid pBM14 contains a unique Bgl II site for cloning of a foreign gene downstream from the $P_L$ promoter. Plasmid pBM11/DP, derived from plasmid pBM11, contains the the nucleotide sequences coding for the first 32 amino acids of the lambda N protein followed by nucleotide sequences coding for the acid cleavable aspartic acid-proline dipeptide. pBM11/DP contains a Nco I site and a Cla I site for cloning a foreign gene downstream of the $P_L$ promoter. Plasmid pBM11/PA, derived from plasmid pBM11/M3 which is an embodiment of a pBM11/M described below, contains the nucleotide sequences coding for a hydrophobic signal sequence followed by Hind III, Sma I and BamH I sites for cloning a foreign gene downstream of the $P_L$ promoter. Plasmid pBM11/M is a derivative of the above-described plasmid pBM11 which contains the ribosomal binding site of the lambda N protein but lacks the first 33 amino acids of the N protein. The DNA sequence coding for the first 33 amino acids of the lambda N protein can be removed by introducing a BamH I restriction site at the beginning of the DNA sequence coding for the first amino acid codon of the lambda N protein. Plasmid pBM11/C is also a derivative of the above-described plasmid pBM11 which contains, however, the E. coli consensus ribosomal binding site and lacks the first 33 amino acids of the lambda N protein.

To evaluate the expression plasmids constructed according to this invention, several foreign genes have been cloned downstream from the lambda $P_L$ promoter including chloramphenicol acetyl transferase (CAT)- in both pBM11 and pBm14; isopenicillin N synthetase (IPS) from Cephalosporium acremonium strain cells, platelet factor 4 (PF-4), human transforming growth factor alpha (TGF-α), a hybrid between TGF-α and VGFA ,VGFa derived from vaccinia growth factor, (TGF-α/VGF), and simian TGF-β in pBM11; TGF-α, PF-4, VGFa, human epidermal growth factor (EGF and hybrid genes including TGF-α/VGF/VGF, TGF-α/VGF/TGF-α, TGF-α/TGF-α/VGF, VGF/TGF-α/TGF-α VGF/VGF/TGF-α, and VGF/TGF-α/VGF in pBM11/DP; and VGFa and EGF in pBM11/PA.

Experimental results illustrated that all plasmids are efficient at expressing the foreign gene cloned.

## BRIEF DESCRIPTION OF THE DRAWINGS

FIG. I illustrates the construction of expression plasmid pBM11CAT.
FIG. II illustrates the construction of expression plasmid pBM14CAT.
FIG. III illustrates the construction of expression plasmid pBM11/DP/VGFa.
FIG. IV illustrates the construction of expression plasmid pBM11/PA/EGF.

## DETAILED DESCRIPTION OF THE INVENTION

In one aspect, this invention is an expression plasmid for expressing foreign genes in a host comprising
(a) a DNA fragment of the plasmid pBR322 containing the origin of replication;
(b) a DNA fragment containing the coding sequence of the temperature sensitive repressor cl derived from bacteriophage lambda cl857S7;
(c) the strong leftward promoter, $P_L$, from bacteriophage lambda;
(d) DNA sequences for a ribosomal binding site comprising DNA sequences for the ribosomal binding site of the lambda N gene and, downstream therefrom, DNA sequences for a member of the group of amino-terminal amino acids selected from the group of:
the first 33 amino acids of the bacteriophage lambda N protein, and
the first 32 amino acids of the bacteriophage lambda N protein followed by aspartic acid and proline;
(e) a selective marker; and
(f) a restriction site for cloning a foreign gene downstream from the $P_L$ promoter.

Examples of the foregoing embodiments are those plasmids designated pBM11/M, pBM11, pBM11/DP and pBM11/PA.

In yet another aspect, this invention is an E. coli bacterial host transformed with the expression plasmid according to this invention.

In still another aspect, this invention is a method for expressing foreign genes comprising
(a) transforming host E. coli bacteria with the expression plasmid according to this invention;
(b) disrupting, e.g. lysing, the host E.coli bacteria from step (a) above; and
(c) recovering the foreign gene expressed in the host E. coli bacteria from step (b) above.

As the selective marker, component (e), there may be used any gene which confers antibiotic resistance. Preferably, for reason of convenience in the construction of the expression plasmids according to this invention, there is employed the genes conferring neomycin or tetracycline resistance.

In the plasmid construction according to this invention, there may be used preferably as the restriction site for cloning a foreign gene downstream from the promoter, $P_L$, component (f), a BamHI, BgIII, NcoI, ClaI, HindIII, or SmaI site.

The activity of the bacteriophage promoter can be repressed by a product of the phage gene cl such that foreign protein, which potentially may be toxic, does not accumulate within the bacteria for a long period of time. The control of the leftward promoter, $P_L$, can be achieved by incorporating the cl gene coding for the thermolabile repressor, preferably cl857S7, into the plasmid, or vector, which allows for the controlled activation of transcription at $P_L$ by temperature. At 30°C, the transcription from $P_L$ is repressed and at 42°C the transcription from $P_L$ is activated. The cloning of a foreign gene downstream from the lambda phage $P_L$ promoter leads to the production of the gene product upon temperature induction.

The utility of the expression plasmid according to this invention to express foreign protein was illustrated by cloning the chloramphenicol acetyl transferase (CAT) gene downstream from the $P_L$ promoter. Experimental results illustrated that CAT was produced upon temperature induction and that no assayable CAT activity was obtained with the non-induced culture.

Although the utility of the expression plasmids according to this invention was illustrated by cloning the CAT gene downstream from the bacteriophage lambda $P_L$ promoter, other foreign genes may be cloned in the expression plasmids according to this invention in the place of the CAT gene. For example, other suitable genes which have been so cloned as a part of this invention, include the following:
1. transforming growth factor alpha (TGF-$\alpha$)
2. VGFa derived from vaccinia growth factor
3. human epidermal growth factor (EGF)
4. platelet factor 4 (PF-4)
5. hybrid gene TGF-$\alpha$/VGF
6. hybrid gene TGF-$\alpha$/VGF/VGF
7. hybrid gene TGF-$\alpha$/VGF/TGF-$\alpha$
8. hybrid gene TGF-$\alpha$/TGF-$\alpha$/VGF
9. hybrid gene VGF/TGF-$\alpha$/TGF-$\alpha$
10. hybrid gene VGF/VGF/TGF-$\alpha$
11. hybrid gene VGF/TGF-$\alpha$/VGF
12. isopenicillin N-synthetase from Cephalosporium acremonium strain C113
13. the mature form of simian TGF-$\beta$

The following examples are illustrative of the present invention and descriptive of preferred embodiments. All parts and percentages are by weight and temperatures are in degrees Celsius unless otherwise indicated.

Example 1. Construction of pBM11CAT (FIG. I)

A. Construction of pBM4

1. Preparation of cI857S7 2.4 Kb Bg1 II fragment.

A 2.4 kb DNA fragment containing the gene sequences coding for bacteriophage lambda cI was isolated by digesting (cleaving) 120 ug of lambda cI857S7 DNA (New England BioLabs) with 170 units of Bgl II (BRL) restriction enzyme by incubating the mixture for 2 hr. at 37°C. The digestion mixture was subjected to electrophoresis on a 1% preparative agarose gel using techniques which are conventional in recombinant DNA technology. The 2.4 kb fragment (base pair 35771 to 38103-- numbering based on Daniel, D. L. et al., pg. 519 in Lambda II edited by Hendrix, R. W., Roberts, J. W., Stahl, F. W. and Weisber, R.) was excised from the gel and subjected to electroelution at 100 V for 4 hr. at room temperature. The resulting eluate was recovered, concentrated, extracted 3 times with an equal volume of phenol:chloroform (1:1). DNA was recovered from the aqueous phase by ethanol precipitation in the presence of 1/10 volume of 3M sodium acetate, pH 5.2. The recovery of the fragment was analyzed by agarose gel electrophoresis.

2. Preparation of partial digest of plasmid pPL-lambda.

Plasmid pPL-lambda (5ug, Pharmacia)--pPL-lambda is a pBR322 derivative containing the lambda leftward promoter $P_L$, the lambda N gene and the termination site for transcription of N ($T_L$) and 2 BamHI sites. Optimal digestion conditions were determined for partial digestion of plasmid pPL- lambda with restriction enzyme BamHI. Plasmid pPL-lambda was digested with 5 units of BamHI by incubating the mixture for 20 min. at 37°C. The digested DNA was separated by agarose-electrophoresis and the full-lengh DNA was recovered by electroelution using techniques which are conventional in the art as described substantially in the isolation of the product from the procedure above.

3. Alkaline phosphatase treatment of linearized pPL-lambda DNA and Ligation.

The linearized $pP_L$-Lambda was treated with alkaline phosphatase to remove the 5' phosphates and ligated to the 2.4 kb Bg1 II fragment of cI857S7 (3 $\mu$l, about 250 ng),in the presence of ligase buffer (500mM Tris-HCl, pH 7.8, 100 mM MgCl$_2$, 200 mM DTT, 10 mM ATP), and T4 DNA ligase (BRL). The resulting reaction mixture was incubated at 12°C for about 15 hr and was used directly for subsequent transformation into competent E. coli HB101 cells.

4. Transformation of E. coli HB101 cells.

E. coli cells were made competent by a modification of a procedure described by D. Hanahan, J. Mol. Biol., 166, 557-580 (1983). A saturated culture of the HB101 cells was diluted 1:200 in Luria Broth supplemented with 20 mM MgCl$_2$ and incubated at 37°C in a gyratory water bath until the culture reaches an optical density (OD, i.e., absorbance) at 550 nanometers, $A_{550}$, of 0.3. Then, 20 ml portion of the culture was harvested by centrifugation at 4°C. The resulting pellet was resuspended in 4 ml of ice cold 50 mM MnCl$_2$/20 mM potassium acetate, pH 6.0 and kept on ice for 15 min. This mixture was centrifuged and the pellet was resuspended in 1.4 ml of solution of 10 mM potassium methanesulfonate, pH 6.2, 100 mM of potassium chloride, 45 mM of MnCl$_2$ 4H$_2$O, 10 mM of CaCl$_2$, 3 mM of hexamine CoCl$_3$, 100 $\mu$l of DMSO, and 100 $\mu$l of 1M DTT. 300 $\mu$l of the treated cells were added to 4 $\mu$l and 6 $\mu$l of the ligation mixture from the procedure of step A4 above. The resulting mixture was placed on ice for 30 min., then incubated at 42°C for 90 sec., and then placed on ice for 90 sec. Then, all of the cells were plated on L-Broth agar plates supplemented with 100 $\mu$g/ml of ampicillin. As there is no rapid and convenient method to screen for bacterial transformants containing the insert, the transformants were screened by isolating the various DNA by the rapid plasmid DNA isolation procedure of Holmes and Quigley reported in Anal. Biochem., 114, 193-198 (1981). Plasmid DNA preparations of recombinants which migrate with the "correct" size were further analyzed by restriction map analysis to determine the orientation of the insert.

B. Construction of pBM8.

Plasmid pBR322 (5μg) was digested with restriction enzyme Pst I (3ul, New England BioLabs) by incubating the reaction mixture for 3 hr at 37°C. The reaction was terminated in a conventional manner by 3 successive extractions using a 1:1 (v:v) phenol:chloroform solution followed by 2 ether extractions. The resulting DNA was precipitated in a conventional manner as described above and completeness of digestion was analyzed by electrophoresis on 0.8% agarose gel. The Pst I digested pBR322 (10 μl) was treated with T4 polymerase (1 μl, BRL) in a conventional manner to convert the 3′ protruding ends to blunt ends. The Hind III site was introduced at the Pst I restriction site of pBR322 by ligation of synthetic phosphorylated HindIII linker (5′-d[CAAGCTTG]) (Pharmacia). The resulting DNA was digested with excess Hind III resulting in two DNA fragments, a 3.58 Kb fragment and a 782 bp fragment. The 3.58 Kb fragment was isolated, ligated, and transformed into competent E. coli HB101. The transformants were analyzed as described above by sizing the Hind III digested DNA on agarose gels.

C. Construction of pBM9.

In a first step, plasmid pBM8 was digested with Hind III and BamHI to yield 2 fragments of DNA. The larger of the 2 fragments, a 3.23 Kb fragment, was isolated by agarose gel electrophoresis and recovered by electroelution according to conventional techniques described above. In a second step, plasmid pNeo (Pharmacia) was digested with HindIII and BamHI to yield a 1.5 Kb fragment containing the gene sequences coding for neomycin resistance. The fragment was isolated and purified using conventional techniques. Then, the 3.23 Kb fragment resulting from the digestion of pBM8 with HindIII and BamH I was ligated to the 1.5 Kb fragment from digestion of pNeo with Hind III and BamH I. The resulting ligation mixture was transformed into competent E. coli HB101 and the transformants were screened as described previously.

D. Construction of pBM10.

Plasmid pBM9 was digested with the restriction enzyme Nde I (New England BioLabs). The 5′ protruding end was converted to blunt end using E. coli DNA Polymerase "Klenow" fragment and ligated to a synthetic phosphorylated EcoR I linker (5′-d[GGAATTCC]-3′) at the filled in Nde I site. The resulting DNA was ligated and transformed into competent E. coli HB101 and the transformants were screened as described previously. The resulting plasmid lost the Nde I site and gained the EcoR1 site and was designated pBM10.

E. Construction of pBM11.

In a first step, plasmid pBM10 was digested to completion with restriction enzymes EcoR I and BamH I. The resulting 2.8 Kb fragment containing the neomycin gene and origin of replication was isolated by agarose gel electrophoresis and recovered by electroelution using conventional techniques as described previously. In a second step, plasmid pBM4 was digested to completion with restriction enzymes EcoR I and BamH I. The 2.84 Kb fragment containing the DNA sequences for the cI gene and lambda $P_L$ promoter and the N ribosomal binding site was isolated and recovered. Then, the so-obtained 2.8 Kb fragment of pBM10 and the 2.84 Kb fragment of pBM4 were ligated together. The resulting ligation mixture was transformed into competent E. coli HB101 and the transformants obtained were screened for the presence of pBM11 as described previously. The cells containing pBM11 were screened further by restriction enzyme analysis with BamH I.

F. Construction of pBM11CAT.

In a first step, plasmid pBM11 was digested with BamH I. In order to prevent self-ligation, the DNA was treated with bacterial alkaline phosphatase to remove 5' phosphate. (Alternatively, calf intestinal alkaline phosphatase may be substituted conveniently for the bacterial alkaline phosphatase mentioned above.) In a second step, plasmid pCM4 (Pharmacia), a pBR322 derivative containing chloramphenicol acetyl transferase (CAT) and its ribosomal binding site, was digested with BamH I. The resulting 779 bp fragment containing the CAT gene and its ribosomal binding sites was isolated and recovered. Then, the linearized pBM11 from the first step above was ligated to the 779 bp fragment of pCM4 from the second step above. The ligation mixture was transformed into competent E. coli HB101. The desired pBM11CAT recombinants were identified by isolating DNAs from neomycin resistant transformants followed by agarose gel electrophoresis. The orientation of the CAT gene in pBM11 was determined by restriction map analysis with the restriction enzymes, EcoR I and Hind III.

Example 2. Construction of pBM14CAT (FIG. II).

A. Construction of pBM12.

Plasmid pBR322 was digested with restriction enzyme Pst I. The resulting 3′ protruding end was converted to a blunt end by treating the digestion product with T4 DNA Polymerase according to conventional techniques as described above in Example 1. Then, synthetic Bgl II linker (5′-d[CAGATCTG]) was phosphorylated and ligated onto the blunt end DNA. The ligation reaction mixture was used to transform competent E. coli HB101. Plasmid DNA preparations were prepared from tetracycline resistant transformants using conventional techniques such as those described in Example 1 and subjected to digestion with Pst I and Bgl II to screen for loss of the Pst I restriction site and addition of the Bgl II restriction site.

B. Construction of pBM13.

Plasmid pBM4, obtained as described in Example 1 above, was digested with Hpa I. The 3′ protruding end was converted to a blunt end and the phosphorylated Bgl II linker added to the blunt end using conventional techniques described above. Similarly, the ligation reaction mixture was used to transform competent E. coli HB101.

C. Construction of pBM14.

Plasmid pBM 12 was digested with Bgl II and EcoR I. The 3.6Kb resulting fragment containing the tetracycline gene and origin of replication was isolated and recovered. Then, plasmid pBM13 was digested with Bgl II and EcoR I. The resulting 2.8 Kb fragment containing the bacteriophage lambda cI857 DNA sequences and the lambda $P_L$ and the N ribosomal binding site was isolated and recovered. The so-obtained 3.6 Kb fragment from pBM12 and 2.8Kb fragment from pBM13 were ligated to form plasmid pBM14. The resulting ligation mixture was transformed into E. coli HB101 and the resulting transformants screened for pBM14 and the putative pBM14 was analyzed further by restriction digest using conventional techniques described previously.

D. Construction of pBM14CAT.

First, plasmid pBM14 was linearized by restriction digestion with Bgl II and the resulting DNA was treated with bacterial alkaline phosphatase to remove 5′ phosphate and thereby prevent self-ligation. Next, the 779 bp fragment containing the CAT gene and its ribosomal binding site was obtained as described in Example 1, part F above. Then, the linearized pBM14 and the 779 bp fragment from above were ligated together, and the ligation mixture was transformed into competent E. coli HB101. Transformants containing pBM14CAT were identified by isolating DNA from tetracycline resistant transformants and sizing the DNAs on agarose gel. The orientation of the CAT gene in pBM14CAT was determined by restriction analysis using the restriction enzyme EcoR I.

The expression plasmids thus obtained were evaluated by measuring CAT activity from cells containing PBM11CATR and pBM14CATR. CAT activity was determined spectrophotometrically as described by R. Rodriques and R. Tait in Recombinant DNA Technology - An Introduction, Addison-Wesley, p. 187, 1983. Table I shows the results of induction of CAT activity of pBM11CATR in E. coli cultured in corn steep liquor and Table II shows the results of induction of CAT activity of pBM11CATR and of pBM14CATR in Luria Broth.

Luria Broth is an enriched medium widely used to culture E. coli at laboratory scale. Corn steep liquor is an inexpensive substitute for Luria Broth. The data from Tables I and II show that for pBM11CATR greater CAT activity is obtained from E. coli cultured in corn steep liquor than in Luria Broth.

## TABLE I

| Induction of pBM111CATR with Corn Steep Liquor (CSL) | | | |
|---|---|---|---|
| Sample | units/ml or u mole/min/ml | mg/ml | u/mg |
| 30°C (1 hr.) | 0.016 | 0.48 | - |
| 30°C (2 hrs.) | 0.018 | 0.80 | - |
| 45°C (1 hr.) | 8.09 | 0.44 | 18 |
| 42°C (2 hrs.) | 13.2 | 0.63 | 21 |

Culture induced at $OD_{550} = 0.64$

1 unit of CAT 1 umole of chloramphenicol (CM) acetylated per minute at 37°C.

## TABLE II

| Induction of pBM11CATR | | | |
|---|---|---|---|
| OD550 | units/ml or u mole/min/ml | mg/ml | u/mg |
| 0.35 | 1.56 | 0.16 | 9.8 |
| 0.44 | 1.69 | 0.275 | 6.2 |
| 0.54 | 1.71 | 0.3 | 6 |
| 0.75 | 2.94 | 0.43 | 6.8 |
| 0.81 | 3.31 | N/D | |
| 0.95 | 5.94 | 0.67 | 8.9 |
| Induction of pBM14CATR | | | |
| OD550 | units/ml or u mole/min/ml | mg/ml | u/mg |
| 0.32 | 1.08 | 0.14 | 7.7 |
| 0.43 | 1.42 | 0.28 | 5.1 |
| 0.54 | 1.07 | 0.24 | 4.4 |
| 0.74 | 1.38 | 0.26 | 5.3 |
| 0.80 | 2.63 | 0.47 | 5.6 |

Optical Density of culture determined prior to temperature shift.
Culture incubated at 42°C for one hour.

Example 3. Construction of pBM11/DP/VGFa

A. Construction of pBM11/DP/VGFA4

1. Preparation of VGF 103a and 104a oligonucleotides.

Synthetic oligonucleotides were designed to complete the amino-terminal region of VGF including an aspartic acid proline dipeptide, as shown below:

```
103a   5' GATCGATCCCATGGACATCCCGGCTATCCGTCTGTGCGGCCCGGAAGGC

           GACGGCTACTGCCTGCATGGTAC 3'


104a   5' CAATGCAGGCAGTAGCCGTCGCCTTCCGGGCCGCACAGACGGATAGCCG

           GGATGTCCATGGGATC 3'
```

Oligonucleotides 103a and 104a were synthesized on an Applied Biosystems Oligonucleotide Synthesizer and purified on an acrylamide gel. The oligonucleotides were phosphorylated at the 5′ end using T4 polynucleotide kinase and then annealed to each other yielding a double stranded 0.072kb DNA fragment with a BamH I overhang at the 5′ end of the gene and a Kpn I overhang at the 3′ end of the gene.

2. Preparation of a Kpn I-BamH I 0.08kb fragment of the VGF gene.

Plasmid PLE TVV WI (20 ug) containing a hybrid growth factor gene TGF/VGF/VGF was digested with 50 units of KpnI and then with 50 units of BamHI. The 0.08kb Kpn I-BamH I fragment of the VGF gene was recovered after electrophoresis on an agarose gel using techniques which are conventional in the art.

3. Preparation of BamH I digested dephosphorylated pBM11

Plasmid pBM11 (20 ug) containing the TGF/VGF gene was digested with BamHI (50 units) and the 5′ phosphates were removed by treatment with calf intestinal alkaline phosphatase (CAP). The 5.65kb plasmid fragment was recovered after electrophoresis on an agarose gel.

4. Ligation and isolation of pBM11/DP/VGFA4

The 0.072kb fragment containing the VGF103a and 104a oligonucleotides, the 0.08kb Kpn I-BamH I VGF fragment and the 5.65kb BamH I digested pBM11 plasmid were ligated together and the mixture was used to transform competent E. coli HB101. The transformants were screened by nucleotide sequencing using the Sanger dideoxy-nucleotide method and clone A4 was identified as being correct. This resulted in a plasmid pBM11/DP containing the VGFA4 gene bounded by an NcoI site at the 5′ end and a BamH I site at the 3′end.

B. Construction of pBM11/DP/VGFa

1. Preparation of VGF 1A and 2A oligonucleotides

Synthetic oligonucleotides were designed to convert a valine residue just downstream of the SphI site in the VGFA4 gene to an arginine residue as is found in vaccinia growth factor.
VGF 1A 5′ CCGTTGCTCTCATGGCTACACTGG 3′
VGF 2A 5′ AATTCCAGTGTAGCCATGAGAGCAACGGCATG 3′
Oligonucleotides 1A and 2A were synthesized on an Applied Biosystems Oligonucleotide Synthesizer and were gel purified as described above. The oligonucleotides were phosphorylated at the 5′ end using T4 polynucleotide kinase and then were annealed to each other yielding a double-stranded 24bp DNA fragment with an SphI overhang at the 5′ end and an EcoRI overhang at the 3′ end.

2. Preparation of SphI digested, dephosphorylated PBM11/DP/VGFA4

Plasmid pBM11/DP/VGFA4 (10ug) was digested with 30 units of SphI and the 5′ phosphates were removed by treatment with half intestinal alkaline phosphatase. The 5 kb plasmid fragment was recovered after electrophoresis on an agarose gel.

3. Preparation of an EcoRI-SphI 70bp fragment of pBM11/DP/VGFA4

Plasmid pBM11/DP/VGFA4 (10ug) was digested with 30 units of EcoRI and then 30 units of SphI. The 70bp fragment was recovered after electrophoresis on an agarose gel.

4. Ligation and isolation of pBM11/DP/VGFa

The 24bp fragment containing oligonucleotides VGF 1A and 2A, the 5 kb SphI fragment and the 70bp EcoRI-SphI fragment of pBM11/DP/VGFA4 were ligated together and the mixture was used to trasform competent E.coli HB101 cells. The transformants were screened by nucleotide sequencing using the Sanger dideoxy-nucleotide method and clone A1 was identified as being correct. This resulted in a plasmid where the VGFa gene is located downstream of 32 amino acids of the lambda N protein and the dipeptide aspartic acid-proline. Treatment of the purified fusion protein with formic acid results in cleavage at the acid labile aspartic acid-proline peptide bond (DP) allowing separation of the VGFa protein from the lambda N amino-terminus. Cleavage is such that the VGFa protein is left with a proline residue at the amino terminus.

Several other pBM11/DP expression plasmids containing such genes as TGF-$\alpha$, PF-4, VGFa, human epidermal growth factor (EGF) and hybrid genes, including TGF-$\alpha$/VGF/VGF, TGF-$\alpha$/VGF/TGF-$\alpha$, TGF-$\alpha$/TGF-$\alpha$/VGF, VGF/TGF-$\alpha$/TGF-$\alpha$, VGF/VGF/TGF-$\alpha$, and VGF/TGF-$\alpha$/VGF, have been constructed by following substantially the procedures described above.

The efficacy of the production of fusion proteins containing foreign gene products in the pBM11/DP expression system and the ability to purify functionally active foreign proteins from the fusion product has been shown using pBM11/DP/VGFa as an example.

Several additional modifications of the expression vectors according to this invention were produced.

One of the goals of the modification scheme was to remove the 100 base pair region immediately downstream of the N gene ribosome binding site and initiation codon and at the same time construct a useful cloning site. This cloning site would allow either foreign genes to be expressed (under the control of the $P_L$ promoter) as "authentic" proteins or allow insertion of other amino-terminal sequences, such as hydrophobic signal sequences, to be expressed under the control of the $P_L$ promoter in order to facilitate expression of foreign genes. To accomplish this, oligonucleotide directed mutagenesis was employed. This method utilized short, synthetic, single stranded DNA sequences which carry the desired mutation. The sequence was annealed to the complementary region of the plasmid which has been made single stranded. Following polymerase and ligase treatment, the heteroduplex closed circular plasmid was transformed into competent E. coli cells. Semi-conservative replication in vivo resolved the heteroduplex into the original and mutant homoduplexes.

Example 4. Construction of pBM11/M

pBM11 (20 ug) was cleaved with restriction enzymes BamH I and Pvu I. After complete digestion, the DNA was electrophoresed through an 0.8% agarose gel and the large fragment (5124 bp) was isolated and recovered by electroelution as described previously. A second sample of PBM11 (10 ug) was digested to completion with Sph I and the 3′ protruding ends converted to blunt ends with T4 DNA polymerase. 0.3 ug samples of the above two DNA'S were mixed together with 37.5 pmol of the following phosophorylated synthetic oligodeoxynucleotide (Pharmacia P-L Biochemical):
5′ AGGAGAATTCATATGGATCCACAA 3′
containing the restriction sites EcoR I, Nde I and Bam H I. The mixture was heated at 100 C for 3 minutes, then sequentially cooled as follows: 30 °C for 30 minutes, 4 °C for 30 minutes, and 0 °C for 10 minutes. The reannealed DNA was subsequently treated with T4 DNA ligase and transformed into E. coli HB101, and neomycin resistant transformants were screened for plasmid containing the three new restriction sites encoded by the synthetic olignuceotide. The resultant plamid, containing additional BamHI, Nde I and EcoRI restriction sites, was designated pBM11/M.

Example 5. Construction of pBM11/M1

Plasmid pBM11/M was digested to completion by restriction enzyme BamH I. The cleaved DNA was electrophoresed through a 0.7% agarose gel and the large fragment (5554 bp) was isolated and recovered by electroelution as described previously. This DNA was religated and transformed into competent E. coli HB101. The resultant plasmid, which lacks the 100 bp region of the lambda N gene down stream of the ribosomal binding and translation initiation site, was designated pBM11/M1. The plasmid contains a Bam H I cloning site for foreign gene(s) and the initiation codon, ATG, located immediately before the BamH I site.

Example 6. Construction of pBM11/M2

A first sample of pBM11 (0.3 ug) cleaved with BamH I and Pvu I and a second sample of pBM11 (0.3 ug) was cleaved with Sph I and then treated with T4 polymerase as described above in Example 4 were mixed together with 3.75 pmol of the following phosphorylated synthetic oligodeoxynucleotide (Pharmacia P-L Biochemical):
5′ AGGAGAATCCAGATGGATCCACAA 3′
containing a BamH I restriction site only. The mixture was heated and cooled as described previously, and treated with T4 DNA ligase and E. coli DNA polymerase Klenow" fragment. The resultant DNA was transformed into competent E. coli HB101, and neomycin resistant colonies were screened for plasmid containing the new BamH I restriction site encoded by the synthetic oligodeoxynucleotide. The resultant plasmid was designated pBM11/M2.

Example 7. Construction of pBM11/M3

Plasmid pBM11/M2 was digested to completion by restriction enzyme BamH I. The cleaved DNA was electrophoresed through a 0.7% agarose gel and the large fragment (5554 bp) was isolated and recovered by electroelution as described previously. This DNA was religated and transformed into competent E. coli HB101. The resultant plasmid, which lacks the 100bp region of the lambda N gene downstream of the ribosomal binding and translation initiation sites, and also lacks the additional EcoR I and Nde I restriction sites contained in pBM11/M and pBM11/M1, was designated pBM11/M3.

Example 8. Construction of pBM11/M4

pBM11 M1 (20 ug) was digested to completion by restriction enzymes EcoRI and BamH I. The DNA was electrophoresed through a 0.7% agrose gel and the large fragment (5544bp) was isolated and recovered by electroelution as described previously. This DNA was ligated to the following pair of phosphorylated synthetic oligodeoxynucleotides (200pmol each) which were heated to 65°C and allowed to cool to 25°C in order to anneal:

**5' AATTCCCATGGGG**

**3'    GGGTACCCCTAG 5'**

which contains a Nco I restriction site. Following ligation, the resultant DNA, which was designated pBM11/M4, was transformed into competent E. Coli HB101, and neomycin resistant colonies were screened for plasmid containing a new Nco I site. The Nco I restriction site was introduced for the cloning of foreign gene (s) as well as supplying the translational initiation codon, ATG.

Example 9. Construction of pBM11/M5

Plasmid pBM11/M4 contains the restriction enzyme site, Nco I, for the insertion of foreign genes. Plasmid pBM11/M5 is identical to pBM11/M4 except the restriction site, Nco I, in the neomycin gene has been removed by site-specific mutagenesis as described previously. The advantage of pBM11/M5 over pBM11/M4 is that the cloning of foreign genes does not require partial digestion of the vector with the restriction endonuclease, Nco I.

By following substantially the procedures described above regarding the pBM11CAT and pBM14CAT plasmids, the following genes were cloned and expressed using the pBM11M vector series according to this invention: PF-4, TGF-α/VGF, h-TGF-α, isopenicillin N-synthetase and simian TGF β. The heterologous proteins expressed were evaluated by sodium dodecyl sulfate/polyacrylamide gel electrohporesis (SDS/PAGE).

Example 10. Construction of pBM11/PA/EGF

A. Construction of pBM11/PA

1. Preparation of oligonucleotides coding for a hydrophobic signal sequence.

Synthetic oligonucleotides were designed to allow insertion of DNA coding for a modified alkaline phosphatase signal peptide and a linker region with 3 cloning sites (Hind III, Sma I and BamH I) into the pBM11 expresion vector downstream of the $P_L$ promoter and N gene ribosomal binding site. The nucleotide sequence was optimized to be as similar as possible to the nucleotide sequence of the amino terminus of the lambda N gene as the lambda N gene sequence has evolved with that of its ribosomal binding site for efficient ribosome initiation and translation. In addition, the second amino acid of the alkaline phosphatase signal sequence, the basic amino acid lysine, which is believed to be important in the proper functioning of the signal sequence was changed to an acidic amino acid, aspartic acid. Changing the lysine to aspartic acid did not affect the cleavage of the signal sequence from the foreign gene product. Futhermore, utilization of the expression vector containing this alteration allowed a different compartmentalization of the highly expressed foreign gene product compared with that ordinarily seen with the alkaline phosphatase signal sequence. The sequences of the oligonucleotides coding for the signal sequence are shown below.

PA1 5' GATCAATCTACAATCGCCCTCGCACTTCTCCCACTGCTGTTCACTCCAGTG

ACAAAAGCTTCCCGG 3'

PA2 5' GATCCCGGGAAGCTTTTGTCACTGGAGTGAACAGCAGTGGGAGAAGTGCGA

GGGCGATTGTAGATT 3'

Oligonucleotides A1 and A2 were synthesized on a Applied Biosystems Oligonucleotide Synthesizer and purified on an acrylamide gel. The oligonucleotides were phosphorylated at the 5′ end using T4 polynucleotide kinase and then annealed to each other yielding a double stranded 0.067kb DNA fragment with a BamHI overhang at each end.

2. Preparation of BamHI digested dephosphorylated pBM11/M

Plasmid pBM11/M3 (20ug) was digested with 30 units of BamH I to linearize the plasmid directly after the N gene ribosomal binding site and the ATG codon for the initiating methionine. The 5′ phosphates were removed by digestion with calf intestinal alkaline phosphatase.

3. Ligation and isolation of pBM11/PA

The 0.067kb PA oligonucleotide fragment was ligated to the linearized pBM11/M3 plasmid and the resulting mixture was used to transform competent E. coli HB101. The transformants were screened by nucleotide sequencing as described above.

B. Construction of pBM11/PA/EGF.

1. Preparation of 0.17kb ECOR I-BamH I fragment of EGF.

Plasmid pBM11/DP/EGF (30ug) was digested with 30 units of EcoR I and then treated with 4 units of Klenow fragment of DNA polymerase to create blunt ends. The DNA was finally digested with 30 units of BamH I and the 0.17kb fragment of the EGF gene was recovered after electrophoresis on an agarose gel. The DNA so purified has a blunted EcoR I site at the 5′ end and a BamH I overhang at the 3′ end.

2. Preparation of 0.5kb Pvu I-Hind III fragment of pBM11/PA.

Plasmid pBM11/PA (18ug) was digested with 30 units of Hind III and then treated with Klenow fragment to blunt the ends. The DNA was then digested with Pvu I and the 0.5kb Pvu I-Hind III (blunt) fragment was recovered after electrophoresis on an agarose gel.

3. Preparation of the 5.2kb Pvu I-BamH I fragment of pBM11/PA

Plasmid pBM11/PA (18ug) was digested with 30 units of Pvu I followed by 30 units of BamH I. The 5.2kb fragment was recovered after electrophoresis on an agarose gel.

4. Ligation and isolation of pBM11/PA/EGF.

The 0.17kb EcoR I (blunt)-BamH I fragment, the 0.5kb Pvu I-Hind III (blunt) fragment, and the 5.2kb Pvu I-BamH I fragment were ligated together and the resulting mixture was used to transform competent E. coli HB101. The transformants were screened using DNA sequencing, as described before.

In yet another modification of the expression vectors according to this invention, a pBM11 plasmid containing the E. Coli consensus ribosomal binding site was produced and designated "pBM11/C." Plasmid pBM11, 30 ug, was digested with 80 ug Pvu I and 192 units BamH I. The resulting two fragments (5.1kb and 0.53kb) were separated by gel electrophoresis and isolated by electroelution. The 0.53kb fragment was then digested with restriction enzyme Hae III. The resulting fragments were separated by gel electrophoresis and the 324bp fragment was isolated by electroelution. To construct pBM11C, the 5.1Kb, the 324bp fragments isolated from plasmid pBM11 and a phosphorylated chemically synthesized linker,

5′ GTAAGGAGGTTTAATATTATG 3′

3′ CATTCCTCCAAATTATAATACCTAG 5′,

were ligated together. The plasmid thus constructed is termed pBM11/C. Plasmid pBM11/C has restriction site, BamH I as the cloning site as well as a unique SspI restriction site within the spacer region of the ribosomal binding site. It has been reported that spacer length affects the efficiency of protein translation. The presence of the Ssp I site allows the length of the spacer region to be changed and also allows for the insertion of other cloning sites for other genes to be expressed.

Modification of plasmid pBM11/C has also been performed. pBM11/C, 10 ug, was digested with restriction enzyme SspI, 45U, and the 5′phosphate was removed by phosphatase. This DNA was ligated to a phosphorylated synthetic Nco I linker CCATGG. This plasmid, termed pBM11C/1, contains 2 Nco I sites, one in the neomycin gene and, one for the cloning of the foreign gene(s). To avoid performing NCo I partial digestion of pBM11/C1 in the cloning of foreign gene (s), the Nco I restriction site in the neomycin gene was removed by site-specific mutagenesis using techniques described previously. This plasmid is termed pBM11/C2.

Using the pBM11/C vectors, some of the genes whose proteins have been expressed are:

1. Isopencillin synthetase
2. PF-4
3. VGF
4. Simian TGF-B (mature form)

We have constructed vectors containing different ribosmal binding sites for the expression of non-fused heterologous proteins since some genes are translated more efficiently with different ribosomal binding sites. For example, two fold more isopenicillin synthetase (IPS) was obtained when IPS was placed under the control of consensus ribosmal binding site versus lambda N gene's ribosomal binding site. Conversely, some genes may be expressed at higher amounts when placed under then control of lambda N gene's ribosmal binding site.

Specific examples of preferred expression plasmids according to this invention, all transformed into E. coli HB101, have been deposited with the American Type Culture Collection and have the identification and ATCC Designation given below:

| IDENTIFICATION | ATCC DESIGNATION |
| --- | --- |
| pBM11 | 67366 |
| pBM14 | 67367 |
| pBM11/PA/VGF | 67417 |
| pBM11/DP/VGFa | 67418 |
| pBM11/PA/EGF | 67419 |
| pBM11/M5 | 67436 |
| pBM11/C2 | 67437. |

14

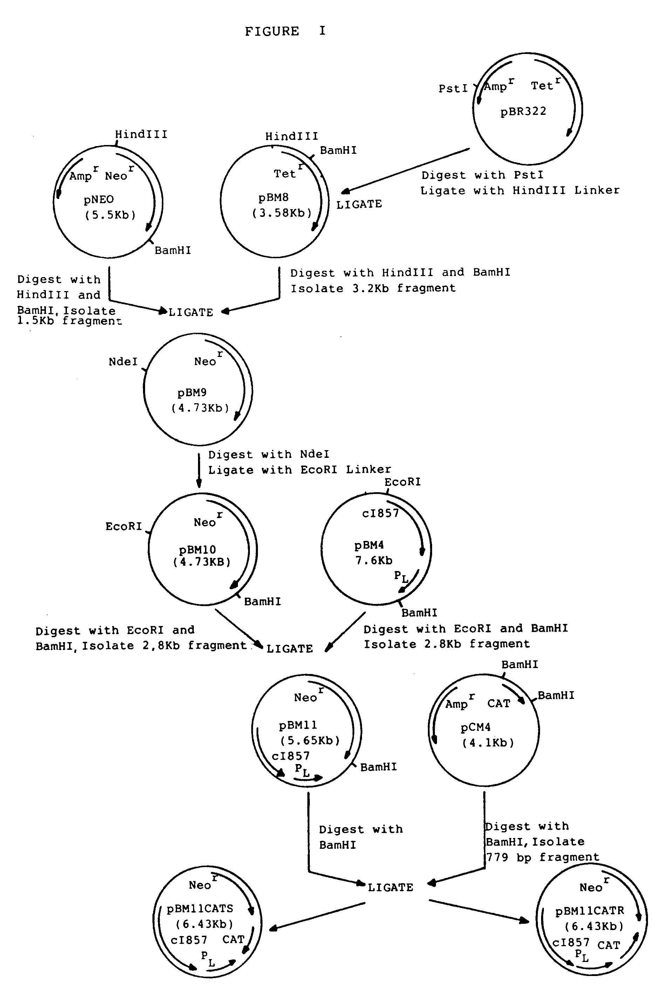

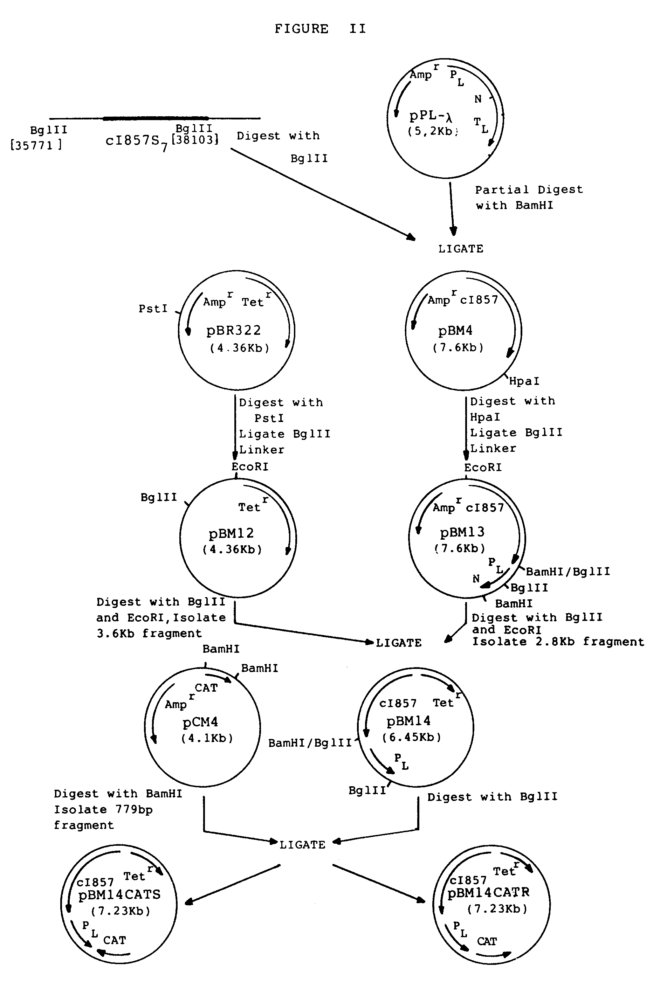

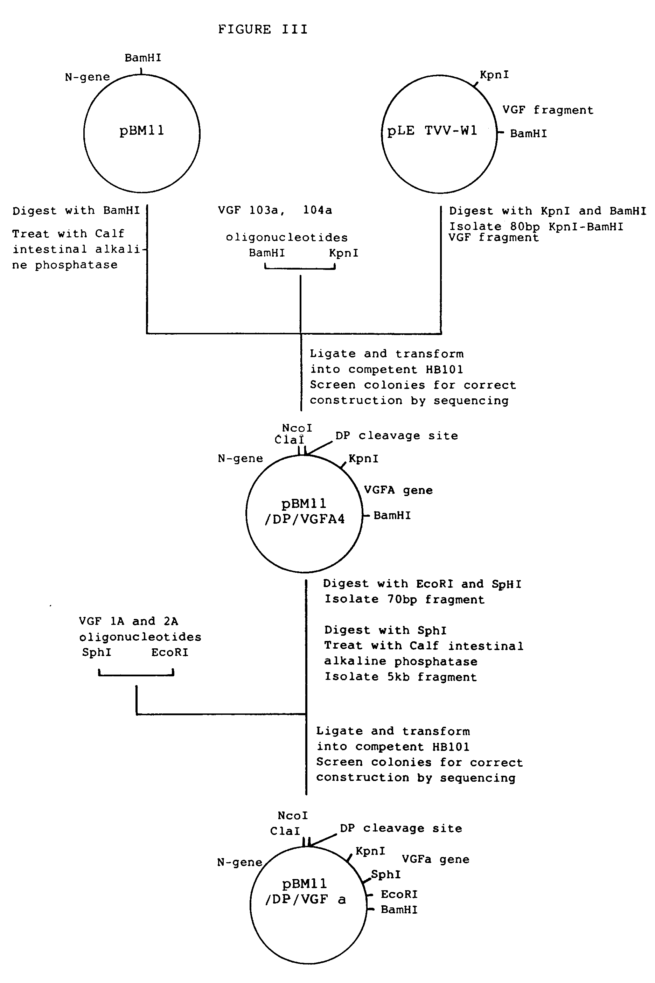

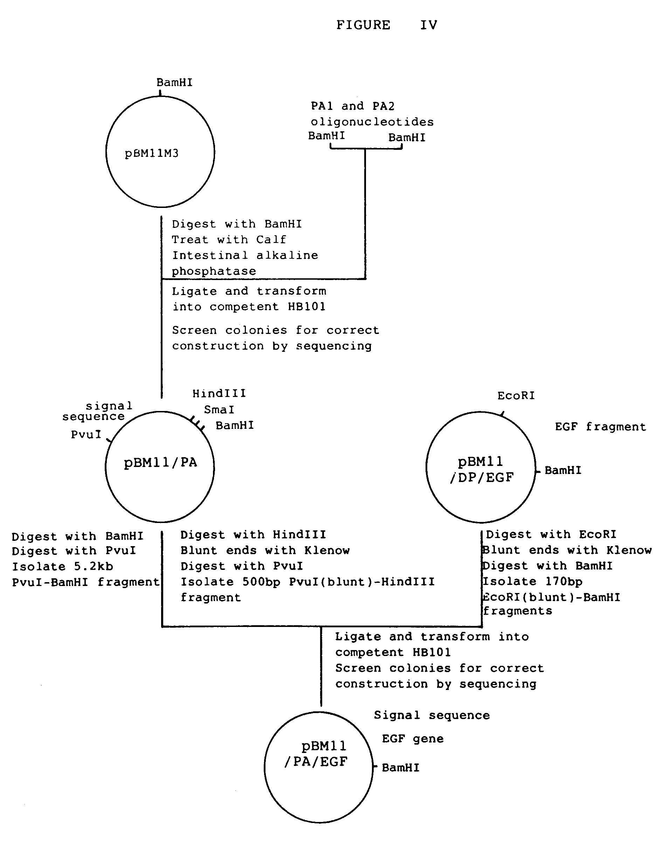

**Claims**

**Claims for the following Contracting State : GR**

1. An expression plasmid for expressing foreign genes in a host comprising:
   (a) a DNA fragment of the plasmid pBR322 containing the origin of replication;
   (b) a DNA fragment containing the coding sequence of the temperature sensitive repressor cI derived from bacteriophage lambda cI857S7;
   (c) the strong leftward promoter, $P_L$, from bacteriophage lambda;
   (d) DNA sequences for a ribosomal binding site comprising DNA sequences for the ribosomal binding site of the lambda N gene and, downstream therefrom, DNA sequences for a member of the group of amino-terminal amino acids selected from the group of:
      the first 33 amino acids of the bacteriophage lambda N protein, and
      the first 32 amino acids of the bacteriophage lambda N protein followed by aspartic acid and proline;
   (e) a selective marker; and
   (f) a restriction site for cloning a foreign gene downstream from the $P_L$ promoter.

2. An expression plasmid according to claim 1 wherein the host is selected from a bacterial host and yeast.

3. An expression plasmid according to claim 2 wherein the host is a bacterial host.

4. An expression plasmid according to claim 3 wherein the bacterial host is E. coli.

5. An expression plasmid according to claim 1 wherein the selective marker is selected from the neomycin resistance gene and the tetracycline resistance gene.

6. An expression plasmid according to claim 1 wherein the restriction site is selected from a BamH I site, and a Bgl II site, a Nco I site, a Cla I site, a Hind III site, and a Sma I site.

7. An expression plasmid according to claim 1 wherein the selective marker is the neomycin resistance gene and the restriction site is an BamH I site.

8. An expression plasmid according to claim 1 wherein the selective marker is the tetracycline resistance gene and the restriction site is a Bgl II site.

9. An expression plasmid according to claim 1 wherein the selective marker is the neomycin resistance gene and the restriction site is a Nco I site.

10. An expression plasmid according to claim 1 wherein the selective marker is the neomycin resistance gene and the restriction site is a Cla I site.

11. An expression plasmid according to claim 1 wherein the selective marker is the neomycin resistance gene and the restriction site is a Hind III site.

12. An expression plasmid according to claim 1 wherein the selective marker is the neomycin resistance gene and the restriction site is a Sma I site.

13. An expression plasmid according to claim 1 wherein the foreign gene is selected from the chloramphenicol acetyl transferase gene, TGF-α/VGF/VGF gene, TGF-α/VGF/TGF-α gene, TGF-α/TGF-α/VGF gene,VGF/TGF-α/TGF-α gene, VGF/VGF/TGF-α, VGF/TGF-α/VGF gene, h-TGF-α gene, h-EGF gene, VGFa gene, simian TGF-$\beta$ gene and isopenicillin N synthetase gene.

14. An E. coli bacterial host transformed with the expression plasmid of anyone of the preceeding claims.

15. A method for expressing foreign genes comprising:
   (a) transforming host E. coli bacteria with an expression plasmid according to claim 1;
   (b) disrupting the host E. coli bacteria from step (a); and

(c) recovering the foreign protein expressed in the host E. coli bacteria from step (b).

16. A method according to claim 15 wherein the foreign gene is selected from the chloramphenicol acetyl transferase gene, TGF-α/VGF/VGF gene, TGF-α/VGF/TGF-α gene, TGF-α/TGF-α/VGF gene, VGF/TGF-α/TGF-α gene, VGF/VGF/TGF-α, VGF/TGF-α/VGF gene, h-TGF-α gene, h-EGF gene, VGFa gene, isopenicillin N synthetase gene and simian TGF-$\beta$ gene.

17. An expression plasmid according to claim 1 further containing a modification wherein the DNA sequences coding for the foreign gene have been removed.

**Claims for the following Contracting State : ES**

1. A method for preparing an expression plasmid for expressing foreign genes in a host which method comprises combining
   (a) a DNA fragment of the plasmid pBR322 containing the origin of replication;
   (b) a DNA fragment containing the coding sequence of the temperature sensitive repressor cl derived from bacteriophage lambda cI857S7;
   (c) the strong leftward promoter, $P_L$, from bacteriophage lambda;
   (d) DNA sequences for a ribosomal binding site comprising DNA sequences for the ribosomal binding site of the lambda N gene and, downstream therefrom, DNA sequences for a member of the group of amino-terminal amino acids selected from the group of:
       the first 33 amino acids of the bacteriophage lambda N protein, and
       the first 32 amino acids of the bacteriophage lambda N protein followed by aspartic acid and proline;
   (e) a selective marker; and
   (f) a restriction site for cloning a foreign gene downstream from the $P_L$ promoter.

2. The method according to claim 1 wherein the host is selected from a bacterial host and yeast.

3. The method according to claim 2 wherein the host is a bacterial host.

4. The method according to claim 3 wherein the bacterial host is E. coli.

5. The method according to claim 1 wherein the selective marker is selected from the neomycin resistance gene and the tetracycline resistance gene.

6. The method according to claim 1 wherein the restriction site is selected from a BamH I site, and a Bgl II site, a Nco I site, a Cla I site, a Hind III site, and a Sma I site.

7. The method according to claim 1 wherein the selective marker is the neomycin resistance gene and the restriction site is a BamH I site.

8. The method according to claim 1 wherein the selective marker is the tetracycline resistance gene and the restriction site is a Bgl II site.

9. The method according to claim 1 wherein the selective marker is the neomycin resistance gene and the restriction site is a Nco I site.

10. The method according to claim 1 wherein the selective marker is the neomycin resistance gene and the restriction site is a Cla I site.

11. The method according to claim 1 wherein the selective marker is the neomycin resistance gene and the restriction site is a Hind III site.

12. The method according to claim 1 wherein the selective marker is the neomycin resistance gene and the restriction site is a Sma I site.

**13.** The method according to claim 1 wherein the foreign gene is selected from the chloramphenicol acetyl transferase gene, TGF-α/VGF/VGF gene, TGF-α/VGF/TGF-α gene, TGF-α/TGF-α/VGF gene, VGF/TGF-α/TGF-α gene, VGF/VGF/TGF-α, VGF/TGF-α/VGF gene, h-TGF-α gene, h-EGF gene, VGFa gene, simian TGF-$\beta$ gene and isopenicillin N synthetase gene.

**14.** A method of preparing a transformed bacterial host, which method comprises the transformation of E. coli with the expression plasmid of anyone of the preceding claims.

**15.** An E. coli bacterial host transformed with the expression plasmid of anyone of the claims 1 to 13.

**16.** A method for expressing foreing genes comprising
(a) transforming host E. coli bacteria with an expression plasmid according to claim 1;
(b) disrupting the host E. coli bacteria from step (a); and
(c) recovering the foreign protein expressed in the host E. coli bacteria from step (b).

**17.** A method according to claim 16 wherein the foreign gene is selected from the chloramphenicol acetyl transferase gene, TGF-α/VGF/VGF gene, TGF-α/VGF/TGF-α gene, TGF-α/TGF-α/VGF gene, VGF/TGF-α/TGF-α gene, VGF/VGF/TGF-α, VGF/TGF-α/VGF gene, h-TGF-α gene, h-EGF gene, VGFa gene, isopenicillin N synthetase gene and simian TGF-$\beta$ gene.

**18.** The method according to claim 1 whereby a plasmid is obtained wherein the DNA sequences coding for the foreign gene have been removed.

**Patentansprüche**

**Patentansprüche für folgenden Vertragsstaat : GR**

**1.** Expressionsplasmid zur Expression von Fremdgenen in einem Wirt, umfassend:
(a) ein DNA-Fragment des Plasmids pBR322, das den Replikationsursprung enthält;
(b) ein DNA-Fragment, das die kodierende Sequenz für den Temperatur-sensitiven cI Repressor, abgeleitet aus dem Bakteriophagen lambda, cI857S7, enthält;
(c) den starken linken Promotor $P_L$ aus dem Bakteriophagen lambda;
(d) DNA-Sequenzen für eine ribosomale Bindungsstelle, umfassend DNA-Sequenzen für die ribosomale Bindungsstelle des lambda N-Gens und, stromabwärts davon, DNA-Sequenzen für ein Mitglied einer Gruppe von aminoterminalen Aminosäuren, ausgewählt unter:
den ersten 33 Aminosäuren des N-Proteins des Bakteriophagen lambda, und
den ersten 32 Aminosäuren des N-Proteins des Bakteriophagen lambda, gefolgt von Asparaginsäure und Prolin;
(e) einen selektierbaren Marker; und
(f) eine Restriktionsschnittstelle für die Klonierung eines Fremdgens, stromabwärts vom $P_L$-Promotor.

**2.** Expressionsplasmid nach Anspruch 1, worin der Wirt ausgewählt ist unter einem bakteriellen Wirt und Hefe.

**3.** Expressionsplasmid nach Anspruch 2, worin der Wirt ein bakterieller Wirt ist.

**4.** Expressionsplasmid nach Anspruch 3, worin der bakterielle Wirt E. coli ist.

**5.** Expressionsplasmid nach Anspruch 1, worin der selektierbare Marker ausgewählt ist unter dem Neomycin-Resistenzgen und dem Tetracyclin-Resistenzgen.

**6.** Expressionsplasmid nach Anspruch 1, worin die Restriktionsschnittstelle ausgewählt ist unter einer BamH I-Stelle, einer Bgl II-Stelle, einer Nco I-Stelle, einer Cla I-Stelle, einer Hind III-Stelle und einer Sma I-Stelle.

**7.** Expressionsplasmid nach Anspruch 1, worin der selektierbare Marker das Neomycin-Resistenzgen und die Restriktionsschnittstelle eine BamH I-Stelle ist.

17

8. Expressionsplasmid nach Anspruch 1, worin der selektierbare Marker das Tetracyclin-Resistenzgen und die Restriktionsschnittstelle eine Bgl II-Stelle ist.

9. Expressionsplasmid nach Anspruch 1, worin der selektierbare Marker das Neomycin-Resistenzgen und die Restriktionsschnittstelle eine Nco I-Stelle ist.

10. Expressionsplasmid nach Anspruch 1, worin der selektierbare Marker das Neomycin-Resistenzgen und die Restriktionsschnittstelle eine Cla I-Stelle ist.

11. Expressionsplasmid nach Anspruch 1, worin der selektierbare Marker das Neomycin-Resistenzgen und die Restriktionsschnittstelle eine Hind III-Stelle ist.

12. Expressionsplasmid nach Anspruch 1, worin der selektierbare Marker das Neomycin-Resistenzgen und die Restriktionsschnittstelle eine Sma I-Stelle ist.

13. Expressionsplasmid nach Anspruch 1, worin das Fremdgen ausgewählt ist unter dem Chloramphenicol-Acetyltransferase-Gen, TGF-$\alpha$/VGF/VGF-Gen, TGF-$\alpha$/VGF/TGF-$\alpha$-Gen, TGF-$\alpha$/TGF-$\alpha$/VGF-Gen, VGF/TGF-$\alpha$/TGF-$\alpha$-Gen, VGF/VGF/TGF-$\alpha$-Gen, VGF/TGF-$\alpha$/VGF-Gen, h-TGF-$\alpha$-Gen, h-EGF-Gen, VGFa-Gen, Simian TGF-$\beta$-Gen und Isopenicillin N Synthetase-Gen.

14. Ein bakterieller E. coli-Wirt, transformiert mit einem Expressionsplasmid gemäß einem der vorhergehenden Ansprüche.

15. Verfahren zur Expression von Fremdgenen, umfassend:
    (a) die Transformation von E. coli-Wirtsbakterien mit einem Expressionsplasmid gemäß Anspruch 1;
    (b) Zerstörung der E. coli-Wirtsbakterien aus Schritt (a); und
    (c) Isolierung des Fremdproteins, das in den E. coli-Wirtsbakterien aus Schritt (b) exprimiert wurde.

16. Verfahren nach Anspruch 15, worin das Fremdgen ausgewählt ist unter dem Chloramphenicol-Acetyltransferase-Gen, TGF-$\alpha$/VGF/VGF-Gen, TGF-$\alpha$/VGF/TGF-$\alpha$-Gen, TGF-$\alpha$/TGF-$\alpha$/VGF-Gen, VGF/TGF-$\alpha$/TGF-$\alpha$-Gen, VGF/VGF/TGF-$\alpha$-Gen, VGF/TGF-$\alpha$/VGF-Gen, h-TGF-$\alpha$-Gen, h-EGF-Gen, VGFa-Gen, Isopenicillin N Synthetase-Gen und Simian TGF-$\beta$-Gen.

17. Expressionsplasmid nach Anspruch 1, aus dem im Rahmen einer weiteren Modifikation die für das Fremdgen kodierenden DNA-Sequenzen entfernt worden sind.

**Patentansprüche für folgenden Vertragsstaat : ES**

1. Verfahren zur Herstellung eines Expressionsplasmids für die Expression von Fremdgenen in einem Wirt, wobei man bei diesem Verfahren miteinander kombiniert:
    (a) ein DNA-Fragment des Plasmids pBR322, das den Replikationsursprung enthält;
    (b) ein DNA-Fragment, das die kodierende Sequenz für den Temperatur-sensitiven cI Repressor, abgeleitet aus dem Bakteriophagen lambda, cI857S7, enthält;
    (c) den starken linken Promotor $P_L$ aus dem Bakteriophagen lambda;
    (d) DNA-Sequenzen für eine ribosomale Bindungsstelle, umfassend DNA-Sequenzen für die ribosomale Bindungsstelle des lambda N-Gens und, stromabwärts davon, DNA-Sequenzen für ein Mitglied einer Gruppe von aminoterminalen Aminosäuren, ausgewählt unter:
        den ersten 33 Aminosäuren des N-Proteins des Bakteriophagen lambda, und
        den ersten 32 Aminosäuren des N-Proteins des Bakteriophagen lambda, gefolgt von Asparaginsäure und Prolin;
    (e) einen selektierbaren Marker; und
    (f) eine Restriktionsschnittstelle für die Klonierung eines Fremdgens, stromabwärts vom $P_L$-Promotor.

2. Verfahren nach Anspruch 1, worin der Wirt ausgewählt ist unter einem bakteriellen Wirt und Hefe.

3. Verfahren nach Anspruch 2, worin der Wirt ein bakterieller Wirt ist.

4. Verfahren nach Anspruch 3, worin der bakterielle Wirt E. coli ist.

5. Verfahren nach Anspruch 1, worin der selektierbare Marker ausgewählt ist unter dem Neomycin-Resistenzgen und dem Tetracyclin-Resistenzgen.

6. Verfahren nach Anspruch 1, worin die Restriktionsschnittstelle ausgewählt ist unter einer BamH I-Stelle, einer Bgl II-Stelle, einer Nco I-Stelle, einer Cla I-Stelle, einer Hind III-stelle und einer Sma I-Stelle.

7. Verfahren nach Anspruch 1, worin der selektierbare Marker das Neomycin-Resistenzgen und die Restriktionsschnittstelle eine BamH I-Stelle ist.

8. Verfahren nach Anspruch 1, worin der selektierbare Marker das Tetracyclin-Resistenzgen und die Restriktionsschnittstelle eine Bgl II-Stelle ist.

9. Verfahren nach Anspruch 1, worin der selektierbare Marker das Neomycin-Resistenzgen und die Restriktionsschnittstelle eine Nco I-Stelle ist.

10. Verfahren nach Anspruch 1, worin der selektierbare Marker das Neomycin-Resistenzgen und die Restriktionsschnittstelle eine Cla I-Stelle ist.

11. Verfahren nach Anspruch 1, worin der selektierbare Marker das Neomycin-Resistenzgen und die Restriktionsschnittstelle eine Hind III-Stelle ist.

12. Verfahren nach Anspruch 1, worin der selektierbare Marker das Neomycin-Resistenzgen und die Restriktionsschnittstelle eine Sma I-Stelle ist.

13. Verfahren nach Anspruch 1, worin das Fremdgen ausgewählt ist unter dem Chloramphenicol-Acetyl-transferase-Gen, TGF-$\alpha$/VGF/VGF-Gen, TGF-$\alpha$/VGF/TGF-$\alpha$-Gen, TGF-$\alpha$/TGF-$\alpha$/VGF-Gen, VGF/TGF-$\alpha$/TGF-$\alpha$-Gen, VGF/VGF/TGF-$\alpha$-Gen, VGF/TGF-$\alpha$/VGF-Gen, h-TGF-$\alpha$-Gen, h-EGF-Gen, VGFa-Gen, Simian TGF-$\beta$-Gen und Isopenicillin N Synthetase-Gen.

14. Verfahren zur Herstellung eines transformierten bakteriellen Wirts, wobei man bei diesem Verfahren E. coli mit einem Expressionsplasmid gemäß einem der vorhergehenden Ansprüche transformiert.

15. Ein bakterieller E. coli-Wirt, transformiert mit einem Expressionsplasmid gemäß einem der Ansprüche 1 bis 13.

16. Verfahren zur Expression von Fremdgenen, umfassend:
    (a) die Transformation von E. coli-Wirtsbakterien mit einem Expressionsplasmid gemäß Anspruch 1;
    (b) Zerstörung der E. coli-Wirtsbakterien aus Schritt (a); und
    (c) Isolierung des Fremdproteins, das in den E. coli-Wirtsbakterien aus Schritt (b) exprimiert wurde.

17. Verfahren nach Anspruch 16, worin das Fremdgen ausgewählt ist unter dem Chloramphenicol-Acetyltransferase-Gen, TGF-$\alpha$/VGF/VGF-Gen, TGF-$\alpha$/VGF/TGF-$\alpha$-Gen, TGF-$\alpha$/TGF-$\alpha$/VGF-Gen, VGF/TGF-$\alpha$/TGF-$\alpha$-Gen, VGF/VGF/TGF-$\alpha$-Gen, VGF/TGF-$\alpha$/VGF-Gen, h-TGF-$\alpha$-Gen, h-EGF-Gen, VGFa-Gen, Isopenicillin N Synthetase-Gen und Simian TGF-$\beta$-Gen.

18. Verfahren nach Anspruch 1, wobei man ein Plasmid erhält, worin die für das Fremdgen kodierenden DNA-Sequenzen entfernt worden sind.


**Revendications**

**Revendications pour l'Etat contractant suivant : GR**

1. Plasmide d'expression pour exprimer des gènes étrangers dans un hôte comprenant :
    (a) un fragment d'ADN du plasmide pBR322 contenant l'origine de réplication;
    (b) un fragment d'ADN contenant la séquence codante du répresseur sensible à la température cl dérivé du bactériophage lambda, CI857S7;
    (c) le promoteur fort à gauche, $P_L$, du bactériophage lambda;
    (d) des séquences d'ADN pour un site de fixation ribosomal comprenant des séquences d'ADN pour le site de fixation ribosomal du gène N de lambda et, en aval des séquences d'ADN pour un membre du groupe d'acides aminés amino-terminaux sélectionnées du groupe :

des 33 premiers acides aminés de la protéine N du bactériophage lambda, et

des 32 premiers acides aminés de la protéine N du bactériophage lambda, suivis de l'acide aspartique et de la proline;

(e) un marqueur sélectif; et

(f) un site de restriction pour cloner un gène étranger en aval du promoteur $P_L$.

2. Plasmide d'expression selon la revendication 1 dans lequel l'hôte est sélectionné parmi un hôte bactérien et une levure.

3. Plasmide d'expression selon la revendication 2 dans lequel l'hôte est un hôte bactérien.

4. Plasmide d'expression selon la revendication 3 dans lequel l'hôte bactérien est E. coli.

5. Plasmide d'expression selon la revendication 1 dans lequel le marqueur sélectif est sélectionné parmi le gène de résistance à la néomycine et le gène de résistance à la tétracycline.

6. Plasmide d'expression selon la revendication 1 dans lequel le site de restriction est sélectionné parmi un site BamH I, et un site Bgl II, un site Nco I, un site Cla I, un site Hind III, et un site Sma I.

7. Plasmide d'expression selon la revendication 1 dans lequel le marqueur sélectif est le gène de la résistance à la néomycine et le site de restriction est un site BamH I.

8. Plasmide d'expression selon la revendication 1 dans lequel le marqueur sélectif est le gène de la résistance à la tétracycline et le site de restriction est un site Bgl II.

9. Plasmide d'expression selon la revendication 1 dans lequel le marqueur sélectif est le gène de la résistance à la néomycine et le site de restriction est un site Nco I.

10. Plasmide d'expression selon la revendication 1 dans lequel le marqueur sélectif est le gène de la résistance à la néomycine et le site de restriction est un site Cla I.

11. Plasmide d'expression selon la revendication 1 dans lequel le marqueur sélectif est le gène de la résistance à la néomycine et le site de restriction est un site Hind III.

12. Plasmide d'expression selon la revendication 1 dans lequel le marqueur sélectif est le gène de la résistance à la néomycine et le site de restriction est un site Sma I.

13. Plasmide d'expression selon la revendication 1 dans lequel le gène étranger est sélectionné parmi le gène de la chloramphénicol acétyl transférase, le gène de TGF-$\alpha$/VGF/VGF, le gène de TGF-$\alpha$/VGF/TGF-$\alpha$ , le gène de TGF-$\alpha$/TGF-$\alpha$/VGF, le gène de VGF/TGF-$\alpha$/TGF-$\alpha$, VGF/VGF/TGF-$\alpha$, le gène de VGF/TGF-$\alpha$/VGF, le gène de h-TGf-$\alpha$, le gène de h-EGF , le gène de VGFa, le gèn simien de TGF-$\beta$ et le gène de l'isopénicilline N synthétase.

14. Hôte bactérien E. coli transformé avec le plasmide d'expression selon l'une quelconque des revendications précédentes.

15. Méthode d'expression de gènes étrangers comprenant :

(a) la transformation d'une bactérie E. coli hôte avec un plasmide d'expression selon la revendication 1;

(b) la destruction de la bactérie E. coli hôte de l'étape (a); et

(c) la récupération de la protéine étrangère exprimée dans la bactérie E. coli hôte de l'étape (b).

16. Méthode selon la revendication 15 dans laquelle le gène étranger est sélectionné parmi le gène de la chloramphénicol acétyl transférase, le gène de TGF-$\alpha$/VGF/VGF, le gène de TGF-$\alpha$/VGF/TGF-$\alpha$, le gène de TGF-$\alpha$/TGF-$\alpha$/VGF, le gène de VGF/TGF-$\alpha$/TGF-$\alpha$ VGF/VGF/TGF-$\alpha$, le gène de VGF/TGF-$\alpha$/VGF, le gène de h-TGF-$\alpha$, le gène de h-EGF, le gène de VGFa, le gène de l'isopénicilline N synthétase et le gène simien de TGF-$\beta$.

**17.** Plasmide d'expression selon la revendication 1 contenant de plus une modification où les séquences d'ADN codantes pour les gènes étrangers ont été éliminées.

**Revendications pour l'Etat contractant suivant : ES**

**1.** Méthode de préparation d'un plasmide d'expression pour exprimer des gènes étrangers dans un hôte laquelle méthode comprend la combinaison de ce qui suit :

(a) un fragment d'ADN du plasmide pBR322 contenant l'origine de réplication;

(b) un fragment d'ADN contenant la séquence codante du répresseur sensible à la température cI dérivé du bactériophage lambda,cI857S7;

(c) le promoteur fort de gauche, $P_L$, du bactériophage lambda;

(d) les séquences d'ADN pour un site de fixation ribosomal comprenant des séquences d'ADN pour le site de fixation ribosomal du gène N de lambda et, en aval, des séquences d'ADN pour un membre du groupe d'acides aminés amino-terminaux sélectionnés dans le groupe :

des 33 premiers acides aminés de la protéine N du bactériophage lambda,

des 32 premiers acides aminés de la protéine N du bactériophage lambda suivis de l'acide aspartique et de la proline;

(e) un marqueur sélectif; et

(f) un site de restriction pour cloner un gène étranger en aval du promoteur $P_L$.

**2.** Méthode selon la revendication 1 dans laquelle l'hôte est sélectionné parmi un hôte bactérien et une levure.

**3.** Méthode selon la revendication 2 dans laquelle l'hôte est un hôte bactérien.

**4.** Méthode selon la revendication 3 dans laquelle l'hôte bactérien est E. coli.

**5.** Méthode selon la revendication 1 dans laquelle le marqueur sélectif est sélectionné parmi le gène de résistance à la néomycine et le gène de résistance à la tétracycline.

**6.** Méthode selon la revendication 1 dans laquelle le site de restriction est sélectionné parmi un site BamH I, et un site Bgl II, un site Nco I, un site Cla I, un site Hind III, et un site Sma I.

**7.** Méthode selon la revendication 1 dans laquelle le marqueur sélectif est le gène de résistance à la néomycine et le site de restriction est un site BamH I.

**8.** Méthode selon la revendication 1 dans laquelle le marqueur sélectif est le gène de résistance à la tétracycline et le site de restriction est un site Bgl II.

**9.** Méthode selon la revendication 1 dans laquelle le marqeur sélectif est le gène de résistance à la néomycine et le site de restriction est un site Nco I.

**10.** Méthode selon la revendication 1 dans laquelle le marqueur sélectif est le gène de résistance à la néomycine et le site de restriction est un site Cla I.

**11.** Méthode selon la revendication 1 dans laquelle le marqueur sélectif est le gène de résistance à la néomycine et le site de restriction est un site Hind III.

**12.** Méthode selon la revendication 1 dans laquelle le marqueur sélectif est le gène de résistance à la néomycine et le site de restriction est un site Sma I.

**13.** Méthode selon la revendication 1 dans laquelle le gène étranger est sélectionné parmi le gène de la chloramphénicol acétyl transférase, le gène de TGF-$\alpha$/VGF/VGF, le gène de TGF-$\alpha$/VGF/TGF-$\alpha$ TGF-$\alpha$/TGF-$\alpha$/VGF, le gène de VGF/TGF-$\alpha$/TGF-$\alpha$, le gène de VGF/VGF/TGF-$\alpha$, le gène de VGF/TGF-$\alpha$/VGF, le gène de h-TGF-$\alpha$, le gène de h-EGF, le gène de VGFa, le gène simien TGF-$\beta$ et le gène de l'isopénicilline N synthétase.

**14.** Méthode de préparation d'un hôte bactérien transformé, laquelle méthode comprend la transformation de E. coli avec le plasmide d'expression de l'une quelconque des revendications précédentes.

**15.** Hôte bactérien E. coli transformé avec le plasmide d'expression de l'une quelconque des revendications 1 à 13.

**16.** Méthode d'expression de gènes étrangers comprenant :
   (a) la transformation de la bactérie E. coli hôte avec un plasmide d'expression selon la revendication 1;
   (b) la destruction de la bactérie E. coli hôte de l'étape (a); et
   (c) la récupération de la protéine étrangère exprimée dans la bactérie E. coli hôte de l'étape (b).

**17.** Méthode selon la revendication 16 dans laquelle le gène étranger est sélectionné parmi le gène de la chloramphénicol acétyl transférase, le gène de TGF-$\alpha$/VGF/VGF, le gène de TGF-$\alpha$/VGF/TGF-$\alpha$, TGF-$\alpha$/TGF-$\alpha$/VGF, le gène de VGF/TGF-$\alpha$/TGF-$\alpha$ , le gène de VGF/VGF/TGF-$\alpha$ le gène de VGF/TGF-$\alpha$/VGF, le gène de h-TGF-$\alpha$, le gène de h-EGF, le gène de VGFa, le gène de l'isopénicilline N synthétase et le gène simien TGF-$\beta$.

**18.** Méthode selon la revendication 1 dans laquelle un plasmide est obtenu où des séquences d'ADN codantes pour le gène ont été éliminées.

FIGURE I

FIGURE II

FIGURE III

FIGURE    IV

BamHI

pBM11M3

PA1 and PA2
oligonucleotides
BamHI          BamHI

Digest with BamHI
Treat with Calf
Intestinal alkaline
phosphatase

Ligate and transform
into competent HB101

Screen colonies for correct
construction by sequencing

signal
sequence

PvuI

HindIII
SmaI
BamHI

pBM11/PA

EcoRI

EGF fragment

pBM11
/DP/EGF

BamHI

Digest with BamHI
Digest with PvuI
Isolate 5.2kb
PvuI-BamHI fragment

Digest with HindIII
Blunt ends with Klenow
Digest with PvuI
Isolate 500bp PvuI(blunt)-HindIII
fragment

Digest with EcoRI
Blunt ends with Klenow
Digest with BamHI
Isolate 170bp
EcoRI(blunt)-BamHI
fragments

Ligate and transform into
competent HB101
Screen colonies for correct
construction by sequencing

Signal sequence

EGF gene

pBM11
/PA/EGF

BamHI

26